# EUROPÄISCHE PATENTSCHRIFT

(11) **EP 0 820 289 B1**
(45) Veröffentlichungstag und Bekanntmachung des Hinweises auf die Patenterteilung: **11.12.2002**
(21) Anmeldenummer: 96907429.3
(22) Anmeldetag: 11.03.1996
(51) Int. Cl.: A61K 31/505, A61P 37/00

(54) **TRAPIDIL ZUR VERWENDUNG IN DER THERAPIE VON IMMUNMODULATORISCH BEEINFLUSSBAREN KRANKHEITSBILDERN**
TRAPIDIL FOR USE IN THE TREATMENT OF IMMUNO-MODULATORILY INFLUENCIBLE CASES
TRAPIDIL POUR UTILISATION DANS LE TRAITEMENT DE SYNDROMES SENSIBLES A L'IMMUNOREGULATION

(30) Priorität: 13.04.1995 DE 19514048
(43) Veröffentlichungstag der Anmeldung: 28.01.1998
(73) Patentinhaber: Rodleben Pharma GmbH, 06862 Rodleben (DE)
(72) Erfinder: WALCH, Hatto, D-88471 Laupheim (DE)
(74) Vertreter: Sandmair, Kurt, Dr. Dr.
(86) Internationale Anmeldenummer: EP9601037
(87) Internationale Veröffentlichungsnummer: WO96032111

(56) Entgegenhaltungen:
- ARZNEIMITTELFORSCHUNG, Bd. 41, Nr. 5, 1991, Seiten 461-468, XP000575960 TH. BETHKE ET AL: "Effects of the triazolopyrimidine trapidil on force of contraction, beating frequency and phosphodiesterase I-IV activity in guinea pig hearts." in der Anmeldung erwähnt
- CIRCULATORY SHOCK, Bd. 44, Nr. 3, 1994, Seiten 97-103, XP000575652 J.C. LEE ET AL.: "Low molecular weight TNF biosynthesis inhibitors: strategies and perspectives."

## Beschreibung

Die Erfindung betrifft die Verwendung von Trapidil (Trapymin) zur Herstellung von Arzneimitteln, die für die Therapie von Erkrankungen geeignet sind, welche sich durch eine immuno-modulatorische Wirkstoffaktivität günstig beeinflussen lassen. Die Substanz Trapidil stellt chemisch ein N,N-Diethyl-5-methyl-s-triazolo[1,5-a]-pyrimidin dar. Seine bislang bekannte klinisch-pharmakologische Aktivität erstreckt sich auf die Behandlung der koronaren Herzkrankheit (KHK), sowohl bei akuten Anfällen als auch deren Prophylaxe und Langzeitanwendung bei dieser chronischen Erkrankung. Das antiischaemisch wirksame Trapidil ist weder hinsichtlich seines klinischen Wirkspektrums noch bezüglich seiner chemischen Struktur mit anderen Koronar-Dilatoren und den übrigen antiangenösen Koronar-Therapeutika des Standes der Technik vergleichbar, wie beispielsweise den Nitraten bzw. "Nitro"-Verbindungen wie dem Nitroglyzerin oder Isosorbiddinitrat, den β-Rezeptorenblockern wie Propranolol oder Calciumantagonisten (Nifedipin).

In Anbetracht seiner Triazolo[1,5-a]-pyrimidin-Struktur weist Trapidil unter den Koronar-Therapeutika eine Ausnahmestellung auf, da es in dieser Reihe von Arzneimitteln den einzigen Wirkstoff mit dieser besonderen Ringstruktur darstellt. Aus der Gruppe der Triazolpyrimidine sind im wesentlichen therapeutisch lediglich das 1,4-Dihydro-5-(2-propoxyphenyl)-7H-1,2,3-triazolo[4,5-d]-pyrimidin-7-on versuchsweise als Antiasthmatikum untersucht, sowie das weniger geläufige 5-Amino1,6-dihydro-7H-1,2,3-triazolo[4,5-d]-pyrimidin-7-on, auch als 8-Azaguanin bekannt, als Zytostatikum bekannt geworden ist.

Aus Untersuchungen am Myokard im Tiermodell (T. Betke, H. Mehl, W. Meier, W. Schmitz, et al.: Effects of the Triazolopyrimidine trapidil on force of contraction, beating frequency and phosphor diesterase I-IV activity guinea pig hearts, Arzneimittelforschung 41/5, S. 461-468, 1991) ist es bekannt, daß Trapidil die Phosphodiesterasen I-IV hemmt, d.h. einen unspezifischen Inhibitor der Isoenzyme PDE I-IV darstellt. Dies steht im Gegensatz zu den spezifischen PDE-Inhibitoren in Form von Methylxanthinen, beispielsweise des Pentoxifyllins, das ein spezifischer Inhibitor des Isoenzyms PDE III ist. Diese Aktivität bedeutet einen antagonistischen Effekt im Hinblick auf den Adenosin-Rezeptor. Das heißt mit anderen Worten, daß Adenosin-Rezeptoren von Trapidil unbeeinfußt bleiben, was durch die Untersuchungen von E. G. Krause, Hemmung der Isoenzyme der Phosphodiesterase aus Herzmuskel, glatter Muskulatur und Thrombozyten durch Trapidil (siehe Trapidil-Workshop, 28./29. Juni 1991 in München) aufgezeigt werden konnte.

Aus Circulatory Shock, 44, Seiten 97 bis 103 (1995) ist es ferner bekannt, daß das Pentoxifyllin im Zusammenhang mit einer TNF-α inhibierenden Aktivität Vorteile bei bakteriellen Infektionen oder der Mäusemalaria haben kann. Es ist auch ganz allgemein bekannt, daß Pentoxifyllin, ein Xanthinderivat, das eine vom Trapidil völlig verschiedene chemische Struktur aufweist, vornehmlich peripher durchblutungsaktive Wirksamkeit besitzt. Somit ist auch die Kreislaufaktivität des Trapidils mit seiner koronaraktiven Wirksamkeit vom Wirkprofil des Pentoxifyllins völlig verschieden.

Im Hinblick auf diese spezifische koronaraktive Wirkung des Trapidils wurde nun überraschend gefunden, daß dieses Triazolopyrimidinderivat eine ganze Reihe von Krankheitsbildern günstig beeinflußt, die auf eine Substanz mit immunomodulatorischen Aktivitäten ansprechen. Dazu gehören eine Reihe von Krankheitsbildern, die bei Infektionen, Neoplasmen und pathoimmunogenen Erkrankungen eine Rolle spielen. Insbesondere hat sich in unerwarteter Weise herausgestellt, daß dem Trapidil eine Tumornekrosefaktor (TNF-α) inhibierende Aktivität zukommt.

Generell stellt der TNF ein Zytokin wie IFN-γ, IL und CSF dar, die von verschiedenen Zelltypen wie Monozyten oder Makrophagen gebildet werden. Dieser Faktor besitzt ein weites Spektrum biologischer Aktivitäten, die in der Antwort des Organismus beispielsweise auf Infektionen, Neoplasmen und pathoimmunogene Erkrankungen eine Rolle spielen. Darunter zählen beispielsweise am Endothel eine erhöhte proagulante Aktivität, Extravasation, Zytokin-(IL-1, IL-8, CSF)-Synthese sowie PAF-Synthese und vermehrte Anzahl an Adhäsionsmolekülen. An den Leukozyten bewirkt der TNF eine Erhöhung der Neutrophilendegranulation, Sauerstoffradikale, der Zytokin-(IL-1, CSF)-Synthese sowie eine Beeinflußung der Prostacyclin E₂-Bildung. Die TNF-Aktivität zeigt sich auch an der Lunge, wo eine erhöhte Kapillarpermeabilität, Ödembildung sowie Adhäsion und auch Aktivierung der Leukozyten zu verzeichnen sind.

Sowohl die Leber ist ein Angriffspunkt des TNF, wo sogenannte Akute-Phase-Proteine vermehrt gebildet werden, als auch die Muskeln, in denen eine Steigerung des Proteinabbaus und Verringerung des Glykogengehalts stattfinden. Am ZNS bewirkt der TNF fiebrige Erscheinungen, begleitet von erhöhter Anorexie und ACTH-Ausschüttung. Aus diesen Aktivitäten läßt sich auch die Schlußfolgerung ziehen, daß der TNF eine zentrale Rolle bezüglich der Resistenz des Organismus gegenüber Fremdorganismen und Tumorzellen einnimmt.

Durch die TNF-inhibierende Aktivität des Trapidils ergeben sich daher unter anderem eine Reihe von neuartigen Indikationen für diesen an sich koronaraktiven Wirkstoff.

Somit lassen sich ganz besonders durch Trapidil solche Krankheitsbilder günstig beeinflussen, die sich im Zusammenhang mit der TNF-Mediatorwirkung im Entzündungsgeschehen herausbilden, z.B. solchen, die von Leukozyten stammenden Zytokinen vom Interleukin-Typ (IL) vermittelt werden.

Die Verabreichung von Trapidil ist infolgedessen angezeigt bei Lungenversagen, unter anderem bei multiplem Organversagen im septischen Schock (ARDS/acquired respiratory distress syndrome), bei kachektischer Lungenerkrankung, Pneumonitis, Peritonitis, bei kachektischen Zuständen im Zusammenhang mit dem HIV-Syndrom und akuter Phase bei der Virusreduplikation, sowie kachektischen Zuständen im Zusammenhang mit Tumoren, bei ZNS-Erkrankungen, beispielsweise im Falle der zerebralen Malaria (clinical outcome) oder auch bei der Multiplen Sklerose zur Verminderung der Krankheitsaktivität bzw. der günstigen Beeinflussung bei MS-Schüben, bei vaskulären Kopfschmerzen (Unterdrückung lokaler TNF-Produktion), bei Kontaktekzem, Psoriasis oder Neurodermitis im Zusammenhang mit der Inhibierung einer lokalen TNF-Genese. Als weitere Indikationen kommen die Sklerodermie, die diabetische Triopathie (Neuropathie, Retinopathie und die Nephropathie), wie auch die Keloide sowie Morphaea in Betracht. Als weitere durch Trapidil günstig zu beeinflussende Erkrankungen sind zu nennen: das Lymphödem, das Myxödem, die Sklerodermie, die Calcinosis cutis, die Kawasaki-Erkrankung, Störungen, welche durch die Ablagerung von Immunokomplexen bedingt sind, wie beispielsweise die rheumatoide Arthritis, der systemische Lupus erythematodes, die Periarterlitis nodosa, Poly- und Dermatomyositis, die diffuse fibrosierende Alveolitis, bestimmte Formen von Glomerulopathie, Lepra, die Trypanosomiasis, chronischaggressive Hepatitis und das Dengue-Fieber, weiterhin das Joop-Buckley-Syndrom, welches ein Immundefizienzsyndrom im Zusammenhang mit IgE-Hyperimmunglobulin darstellt, ferner Xantomatosen oder die Sarkoidose.

Die oben erwähnte immuno-modulatorische Aktivität steht auch in Zusammenhang mit der unerwartet gefundenen Interleukininhibierenden Wirkung. Soweit noch nicht unter den vorstehenden neuen medizinischen Indikationen des Trapidils berücksichtigt, hat sich auch eine vorteilhafte Beeinflußbarkeit durch diesen Wirkstoff bei den folgenden Krankheitsbildern abgezeichnet: Appetitverlust, Dialyseschäden, wie beispielsweise die Dialyse-Arthropathie, die Dialyse-Osteopathie oder sonstige Dialyseunverträglichkeiten oder -schäden, endotoxischer Schock, toxisches Schocksyndrom, Kachexie, infektionsbedingte Myalgien, Osteoporose, rheumatische Arthritis, der Gouty-Arthritis, sowie Morbus Crohn und Colitis ulcerosa.

Transplantationsbedingte GVH-Reaktionen lassen sich durch Trapidil im Zusammenhang mit Knochenmarkübertragung ebenfalls günstig beeinflussen. Dies gilt auch bei Nierentransplantationen bezüglich der Abschwächung von OTK-3-Nebenwirkungen durch Gaben dieses Wirkstoffes.

Es sind jedoch auch durch physische Traumen verursachte Läsionen, sei es mechanischen Ursprungs, wie beispielsweise Lazerationen, Kontussionen oder Verwundungen, sei es durch thermische Einwirkungen bedingte Verletzungen der Körpergewebe, wie beispielsweise bei Verbrennungen oder Erfrierungen, oder auch strombedingte Traumen mit Trapidil therapierbar. Das gleiche gilt für die schädlichen Auswirkungen von Bestrahlungen, sei es der Röntgen-, UV-, Infrarot- oder Gamma-Bestrahlung.

Durch die Behandlung mit Trapidil enthaltenden Arzneimitteln lassen sich auch jegliche Arten von Infektionskrankheiten, welche durch Bakterien, Bazillen, Pilze, Viren oder Parasiten hervorgerufen wurden, günstig beeinflussen. Darüber hinaus läßt sich eine Besserung von Symptomen erzielen, die von der schädlichen Einwirkung von Umweltgiften herrühren, beispielsweise durch Schwermetalle, Asbest, Beryllium-, Chrom- oder Quecksilberverbindungen wie auch durch Herbizide und Pestizide bedingte Intoxikationen.

Insbesondere eignet sich das Trapidil aufgrund der obenstehend erwähnten TNF-α-inhibierenden Aktivität zum Einsatz bei einer Therapie mit Arzneistoffen, welche die Freisetzung des Tumornekxosefaktors (TNF) verursachen. Es lassen sich dadurch die Nebenwirkungen derartiger TNF-freisetzenden Substanzen, beispielsweise aus der Reihe der Makrolid- und Polyenantibiotika mit zum Teil antimykotischer Aktivität unterdrücken, insbesondere solchen, die aus Streptomycesarten, wie z. B. Streptomyces nodosus, S. noursei oder S. antibioticus gewonnen wurden. Es ist im Falle der Verabreichung von Cyclosporin A eine Abschwächung der Nephrotoxizität durch zusätzliche Gaben von Trapidil möglich.

Somit wird durch diese Kombinationtherapie eine in toxikologischer Hinsicht ungefährliche Behandlung ermöglicht. Das Trapidil selbst läßt sich auch direkt mit dem Tumornekrosefaktor TNF beispielsweise bei der Therapie von Neoplasmen unter Vermeidung einer akuten Lungenschädigung und Hypotension verwenden. Die Verabreichung von Trapidil kommt auch im Falle eines akuten Nierenversagens nach Reperfusion der Niere in Betracht.

Es hat sich auch herausgestellt, daß das Trapidil eine günstige Wirkung bei der Behandlung proliferativer Hauterkrankungen, beispielsweise der atopischen, unspezifischen oder allergischen Kontaktdermatitis, bei basalen oder schuppigen Hautkarzinomen, bei Ichthyosis-Erkrankungen, Hyperkeratosen, prämaligner Sonnenkeratose oder anderen Keratosen, Akne, sowie seborrhöischer Dermatitis entfaltet.

In ganz unerwarteter Weise lassen sich auch neurologische Erkrankungen wie Morbus Parkinson oder Epilepsie durch Trapidil günstig beeinflussen.

Die zur Therapie oder Prophylaxe eingesetzten Einzeldosen des Trapidils betragen zwischen 10 mg und 500 mg, die täglich verabreichte Gesamtmenge liegt zwischen 50 mg und 3000 mg bei oraler Gabe bzw. bei 1 mg bis 10 mg pro Kilogramm Körpergewicht intravenös und 250 bis 3000 mg topisch pro Einzelapplikation. Die bevorzugten Dosierungen betragen bei oraler oder parenteraler Verabreichung 100 mg bis 600 mg täglich, als Einzeldosierung sind insbesondere 50 mg, 100 mg, 200 mg, 300 mg, 400 mg oder 500 mg bevorzugt.

Die gleichen Dosierungen in Form der Einzeldosen und der Tagesdosen kommen auch bei der Kombination des Trapidils mit anderen Wirkstoffen in Betracht. Eine der Kombinationsmöglichkeiten des Trapidils besteht in der gemeinsamen oder getrennten Verabreichung in Verbindung mit Corticosteroiden, welche in einer Einzeldosierung zwischen 0,01 mg bis 300 mg zur Anwendung gelangen, je nach dem verwendeten Typ an Glucocorticoid, wie beispielsweise Prednisolon, Prednison, Hydrocortison (Cortison), Dexamethason, Cortison, Triamincolon, Betamethason, kommen Einzeldosierungen von 0,5 mg, 1 mg, 2 mg, 5 mg, 10 mg, 50 mg, 100 mg, 200 mg oder 500 mg in Betracht, wobei die Tagesdosen den zweifachen, dreifachen oder vierfachen Wert erreichen und zwischen 1 mg und 1000 mg betragen können.

Andere vorteilhafte Kombinationen ergeben sich aus der gemeinsamen oder separaten Verabreichung des Trapidils zusammen mit Interferonen, beispielsweise dem α-, β-, oder γ-Interferon vom Typ I, welches in einer Menge von 0,1 bis 100 x 10⁶ I.E. verabreicht wird. Als bevorzugte Einzeldosierung des Interferons sind 1, 3, 5, 10 oder 20 x 10⁶ I.E. pro Applikation bzw. die zweifache, dreifache oder vierfache Menge als Tagesdosierung vorgesehen. Sofern das Trapidil zur Unterdrückung von TNF-induzierten Nebenwirkungen eingesetzt werden soll, kommen dabei die obenstehenden Einzel- bzw. Tagesdosierungen zum Einsatz, wobei der TNF-bedingte Schädigungen verursachende Arzneistoff in der üblichen Menge von z. B. 0,1 mg bis 1 mg pro Kilogramm Körpergewicht täglich bzw, als Einzeldosis 50 mg Lyophilisat für die Infusion im Falle eines Makrolid- bzw. Polyenantibiotikums oder -antimykotikums verabreicht wird.

Bei der Therapie von Neoplasmen wird das Trapidil ebenfalls in den obengenannten Tages- oder Einzeldosierungen angewandt, wobei im Falle der direkten Verwendung des Tumornekrosefaktors TNF selbst die Dosierung des letztgenannten Wirkstoffs zwischen 0,5 und 10 x 10⁶ I.E. pro Kilogramm Körpergewicht einzeln bzw. täglich beträgt.

Die Herstellung der Arzneiformen, sowohl der Monosubstanzpräparate mit einem Gehalt an Trapidil, als auch die Kombinationspräparate, die Trapidil zusammen mit anderen wirkstoffen enthalten, insbesondere einen oder mehrere der obenstehend erwähnten Arzneistoffe, erfolgt in herkömmlicher Weise. Die jeweiligen Einzeldosierungen können im Falle der Kombinationspräparate sowohl je nach Verträglichkeit der Wirkstoffe untereinander unmittelbar kombiniert sein als auch in einer Arzneimittelpackung in getrennter Form voneinander vorliegen. Dadurch kann ihre Anwendung entweder durch Einzelverabreichung nebeneinander oder in der getrennten Form in aufeinanderfolgenden Verabreichungen erfolgen. Die letztgenannte Möglichkeit erstreckt sich insbesondere auf solche Arzneiformen, bei denen die gemeinsame Verabreichung der verschiedenen Wirkstoffe zu chemischen oder pharmakologischen Inkompatibilitäten führen würde oder der Verabreichung aufgrund der speziellen pharmakokinetischen Bedingungen unterschiedlich zu wählen ist; beispielsweise kann in diesen Fällen der eine Wirkstoff peroral, der andere Wirkstoff parenteral, gegebenenfalls in zeitlicher Verschiebung, verabreicht werden.

Für die perorale Verabreichung kommen Tabletten, Dragees, Retardtabletten, Kapseln, Filmtabletten, Pulver, Kaugummi oder flüssige Formen, wie beispielsweise Suspensionen, Elixiere oder Lösungen in Betracht. Für die muköse Verabreichung eignen sich auch sublinguale Formen, beispielsweise Tabletten oder Kaugummi, etc. Eine andere Form der mukösen Verabreichung besteht in der Anwendung von Suppositorien. Die parenterale Verabreichung kann auf intravenösem oder interarteriellem Wege erfolgen. Dabei kommen wäßrige Injektions- oder Infusionslösungen in Betracht, die mittels Sterilfiltration hergestellt werden. Für die topisch-systemische Applikation des Trapidils lassen sich auch transdermale Arzneiformen einsetzen, beispielsweise Pflaster.

| Retardtablette | |
|---|---|
| Zusammensetzung Inhaltsstoffe: | mg/Tablette: |
| Trapidil | 300,00 |
| Natriumalginat | 150,00 |
| Laktose·1H₂O | 110,00 |
| Poly-(1-vinyl-2-pyrrolidion) | 20,00 |
| gereinigtes Wasser | 23,20 |
| Talkum | 10,80 |

Das Natriumalginat kann durch andere hydrophile Gerüstbildner wie Cellulosederivate (z. B. Hydroxyethylcellulose, Hydroxypropylcellulose, Methylhydroxypropylcellulose, Polyacrylsäure und/oder Polyvinylalkohol ersetzt werden.

Die Dosierung des Wirkstoffes läßt sich je nach den Bedürfnissen des Patienten innerhalb des oben erläuterten Rahmens unter entsprechender Anpassung der Hilfsstoffsmengen variieren. In Einzelfällen kann aber die Einzeldosis jeweils bis zu 700, 800, 900, 1000, 1100 oder 1200 mg bei der Retardform betragen.

Als geeignete Hilfsstoffe für beliebige feste und flüssige Arzneiformen kommen Cellulose bzw. Cellulosederivate, Gelatine, Stärke oder Zucker wie Laktose, Saccharose, Sorbit, Mannit oder wasserunlösliche anorganische Stoffe, wie beispielsweise Dicalciumphosphat oder Calciumphosphat, Glucose, Zerfallsmittel (bzw. Sprengmittel), Emulgier-, Dispergieroder Suspendiermittel sowie Bindemittel, wie beispielsweise Natriumcarboxymethylstärke, Carboxymethylcellulose, Calcium oder andere Cellulosederivate, Alginsäure, Polyvinylpyrrolidone, Gelatine, Stärke, Alginate oder Polyethylenglykole (Polywachse) oder Gleit- und Gegenklebemittel in Betracht. Erforderlichenfalls werden auch Stabilisatoren, Farb- oder Geschmacksstoffe, Konservierungsmittel oder Puffersubstanzen zur Herstellung der Isotonie und chemischen Stabilität hinzugefügt. Es lassen sich auch verschiedene Hilfsstoffe zur Steuerung einer gezielten Freisetzungsrate bei festen oralen Arzneiformen oder eine jeweils geeignete Korngroßenverteilung der Wirkstoffe, welche ein bestimmtes Freisetzungsbild ergeben, zum Einsatz bringen.

Für die topische Verabreichung eignen sich Salben, Cremes von O/W sowie W/O und amphiphile Systeme, Mikroemulsionen sowie Gele oder Pasten und Lotionen. Bei der Herstellung dieser äußerlich verabreichbaren Formen werden übliche Hilfsstoffe wie hautverträgliche Lösungsmittel, wie Wasser, Ethanol oder Isopropanol verwendet, ferner mehrwertige Alkohole wie Propylenglykol oder Glycerin. Als konsistenzgebende Stoffe oder Solubilierungsmittel kommen Celluloseether, längerkettige Fettalkohole sowie Ethoxylierungsprodukte, langkettige Fettsäuren, hydriertes Erdnußöl, halbfeste Lipide wie Wollwachs, Paraffinöl oder Paraffin fester oder halbfester Konsistenz, Ester der Öl-, Palmitin- und/oder Myristinsäure und halbsynthetische mittelkettige Triglyceride, Polyglykole oder Vaseline in Betracht. Als Emulgatoren bzw. Tenside eignen sich unter anderem Verbindungen aus der Reihe partiell mit langkettigen Fettsäuren veresterte mehrwertige Alkohole (vom Typ Glycerinmonostearat), polyoxyethylierte Derivate dieser Verbindungen (vom Typ Tagat^{(R)} S2), Sorbitanfettsäureester (vom Typ Span^{(R)}, Arlacel^{(R)}), polyoxyethylierte Derivate dieser Verbindungen (vom Typ Polysorbat = Tween^{(R)}), Fettsäureester des Polyoxyethylentyps (Typ Myrj^{(R)}) und Fettalkoholester des Polyoxyethylens (Typ Brij^{(R)}).

Die im Vorstehenden aufgezeigte neue Trapidilwirksamkeit wurde anhand von Tests zur Bestimmung der Zytokin-Freisetzung und Messung der Aktivität von TNF- aufgefunden. Die quantitative Bestimmung der Zytokin-Freisetzung erfolgte in einem Bioassay unter Verwendung des WEHI 164 Subklon 13 (Fibrosarkom lysierender Assay), wie er in Espevik T. & Nissen-Meyer J.: "A highly sensitive cell line, WEHI 164 clone 13, for measuring cytotoxic factor/tumor necrosis factor from human monocytes", J. Immunol. Methods, 95, (1986), S. 99ff beschrieben ist. Dabei werden Proben von Reinverdünnungen mit den Hybridomzellen 20 Stunden lang vor der Durchführung des MTT-Tests inkubiert, wie aus der Publikation Hanssen M. B., Nielsen S. E. & Berg K.: Reexamination and further development of a precise and rapid dye method for measuring cell growth/cell kill. J. Immunol. Methods, 119, (1989), S. 203ff ersichtlich ist. Die Kulturplatten wurden danach in einem Mikro-Elisa-Meßgerät (MR 580, Dynatech Laboratories Inc. Alexandria, VA, U.S.A.) bei 540 nm gemessen. Die so ermittelten Meßwerte an gebildetem TNF-α wurden unter Benutzung eines internen Standards von humanem, rekombinantem TNF-α (Boehringer, Mannheim, BRD) in pg/mm ausgedrückt.

Ein weiterer Test bestand darin, residente peritoneale Makrophagen der Maus in einer Menge von 2 x 10⁵ in 0,2 ml Iscove-Medium mit den Prüfsubstanzen 20 Minuten lang vorzuinkubieren. Dann wurde die Zytokinbildung durch Inkubation mit Lipopolysaccharid in einer Menge von 100 ng/ml aus Salmonella abortus equi für eine Zeitdauer von 4 Stunden induziert. Anschließend wurde der TNF in dem Cytotoxizitätsbestimmungsverfahren auf Basis von WEHT-Zellen quantitativ bestimmt. Nach diesen Vorbereitungen konnte die Inhibierung durch Trapidil in Konzentrationen zwischen 1 und 10 000 µmol systematisch durchgeführt werden. Es stellte sich dabei überraschenderweise die dosisabhängige Hemmung der TNF-Synthese durch Trapidil heraus. Dieser in vitro-Befund konnte auf die klinischen in vivo-Verhältnisse übertragen werden.

## Patentansprüche

1. Verwendung von Trapidil zur Herstellung eines Arzneimittels zur Therapie von Krankheitsbildern, die auf Substanzen mit immunmodulatorischen Aktivitäten ansprechen.

2. Verwendung von Trapidil nach Anspruch 1, **dadurch gekennzeichnet, daß** Arzneimittel hergestellt werden, die bei der Therapie von durch die TNF-α inhibierende Aktivität des Trapidils beeinflußbare Krankheitsbilder verwendet werden.

3. Verwendung von Trapidil zur Herstellung eines Arzneimittels zur Therapie von Krankheiten gemäß Anspruch 1 oder 2, die im Zusammenhang mit von Leukozyten stammenden Zytokinen vom Typ Tumornekrosefaktor (TNF) stehen.

4. Verwendung gemäß den Ansprüchen 1 bis 3, **dadurch gekennzeichnet, daß** die Dosiereinheiten des Trapidils in der Arzneiform zwischen 10 mg und 500 mg betragen.

5. Verwendung gemäß den Ansprüchen 1 bis 4, **dadurch gekennzeichnet, daß** zusätzlich zu dem Trapidil noch eine Verbindung vom Interferon-Typ gemeinsam oder in separater Verabreichung kombiniert zu applizieren ist.

6. Verwendung gemäß den Ansprüchen 1 bis 4, **dadurch gekennzeichnet, daß** zusätzlich zu dem Trapidil noch ein die TNF-Produktion fördernder Wirkstoff gemeinsam oder separat nebeneinander zum Einsatz gelangen soll.

7. Verwendung gemäß den Ansprüchen 1 bis 4, **dadurch gekennzeichnet, daß** kombiniert neben dem Trapidil noch ein Glucocorticoid gemeinsam oder in separater Verabreichung zu applizieren ist.

## Claims

1. The use of Trapidil for preparing a drug for the therapy of sydromes which respond to substances with immunomodulatory activity.

2. The use of Trapidil according to claim 1, **characterised in that** drugs are prepared which are used for the therapy of syndromes which may be influenced by the TNF-α-inhibiting activity of Trapidil.

3. The use of Trapidil for preparing a drug for the therapy of diseases according to claim 1 or 2 which are associated with leukocyte-derived cytokins of the tumour necrosis factor (TNF) type.

4. The use according to claim 1 to 3, **characterised in that** the Trapidil dosage units in drug form are between 10 mg and 500 mg.

5. The use according to claim 1 to 4, **characterised in that** a compound of the interferon type is to be applied additionally in combination with the Trapidil either jointly or by separate administration.

6. The use according to claim 1 to 4, **characterised in that** an active ingredient promoting TNF production is to be used in addition to the Trapidil either jointly or separately side by side.

7. The use according to claim 1 to 4, **characterised in that**, in addition to the Trapidil, a glucocorticoid is to be applied in combination therewith either jointly or by separate administration.

## Revendications

1. Utilisation de trapidil pour fabriquer un médicament destiné au traitement de tableaux cliniques qui sont sensibles à des substances ayant une activité immunomodulatrice.

2. Utilisation de trapidil selon la revendication 1, **caractérisée en ce qu'**il est fabriqué des médicaments, qui sont utilisés lors du traitement de tableaux cliniques pouvant être influencés par l'activité inhibitrice du TNF-α présentée par le trapidil.

3. Utilisation de trapidil pour fabriquer un médicament destiné au traitement de maladies selon la revendication 1 ou 2, qui sont liées à des cytokines, provenant de leucocytes, du type facteur de nécrose tumorale (TNF).

4. Utilisation selon les revendications 1 à 3, **caractérisée en ce que** les unités posologiques du trapidil dans la forme pharmaceutique sont comprise entre 10 et 500 mg.

5. Utilisation selon les revendications 1 à 4, **caractérisée en ce que**, en plus du trapidil, il convient aussi d'appliquer en combinaison un composé du type interféron, par une administration simultanée ou distincte.

6. Utilisation selon les revendications 1 à 4, **caractérisée en ce que**, outre le trapidil, on doit encore utiliser un principe actif favorisant la production du TNF, simultanément ou séparément l'un de l'autre.

7. Utilisation selon les revendications 1 à 4, **caractérisée en ce que**, outre le trapidil, on peut administrer encore, en combinaison, un glucocorticoïde par une administration simultanée ou distincte.
